# EUROPEAN PATENT SPECIFICATION

(11) **EP 2 724 664 B1**
(45) Date of publication and mention of the grant of the patent: **11.02.2015**
(21) Application number: 13182775.0
(22) Date of filing: 03.09.2013
(51) Int. Cl.: A61B 5/00, A61B 5/024, A61B 5/0404, A61B 5/11, A61B 5/145

(54) **Portable electrocardiograph**
Tragbarer Elektrokardiograph
Électrocardiographe portable

(30) Priority: 29.10.2012 IT MI20121834
(43) Date of publication of application: 30.04.2014
(73) Proprietor: Biotechware S.r.l., 10129 Torino (IT)
(72) Inventor: Sappia, Alessandro, 10152 Torino (IT)
(74) Representative: Fiorentino, Luca

(56) References cited:
- US-A- 3 848 582
- US-A1- 2003 226 695
- US-A1- 2010 261 979
- US-A1- 2011 015 496

## Description

The present invention refers to a portable electrocardiograph, in other words, to a portable device for recording electrocardiograms.

Devices for detecting the trend of the heart-beat of a patient are known.

The electrocardiograph, by means of electrodes, is capable of detecting the trend of heart-beats of a patient, which are recorded and displayed in a print format and/or on a screen which the electrocardiograph is provided with. Therefore, the data can be downloaded and transferred for example to an outside computer.

The portable electrocardiographs are for example used on ambulances, rescue helicopters, pharmacies. Electrocardiographs are provided with a handle which enables them to be gripped and supported in order to make them easier to transport.

However, such known electrocardiographs are difficult to be used. Indeed, it is not easy to handle and support them. For example, when it is necessary to input data, for example by a keyboard incorporated in the electrocardiograph itself, the operation is complicated by the fact it is necessary to simultaneously support the electrocardiograph and write by the keyboard. Further, often, the electrocardiograph is used by the same patient, which requires him/her to input his/her data (first name, family name, tax code number) in an uncomfortable position, for example laid in a bed. This operation therefore can be rather complex particularly for a patient, for the above mentioned reasons.

Electrocardiographs according to the prior art are disclosed in documents US 3 848 582 A, US 2010/261979 A1, US 2001/015496 A1 and US 2003/226695 A1.

Therefore, it is an object of the present invention to make available a portable electrocardiograph which is easy to transport and convenient to use, both by the operators (doctors, paramedics or nurses) and by patients. Specifically, the object of the present invention consists of making available an electrocardiograph which can be simply useable (both for writing or reading).

This and other objects can be obtained by a portable electrocardiograph according to claim 1.

To better understand the invention and appreciate its advantages in the following some exemplifying non limiting embodiments thereof will be described with reference to the attached figures, wherein:
Figure 1 is a perspective view of a first side of an electrocardiograph according to the invention;
Figure 2 is a perspective view of a second side of the electrocardiograph of Figure 1;
Figure 3 is a perspective view of a first side of a table provided with a gripping-and-supporting member of the electrocardiograph of Figure 1;
Figure 4 is a perspective view on a second side of the table of the electrocardiograph of Figure 3;
Figure 5 is a plan view of a table of the electrocardiograph according to the invention;
Figure 6 is a side view of the table of Figure 5;
Figure 7 is a perspective view of the partially disassembled electrocardiograph of Figure 1.

Referring to the figures, a portable electrocardiograph is indicated by reference 1. Electrocardiograph is mainly intended to perform measurements of the heart-beat of a patient. The portable electrocardiograph 1 can be used for example in ambulances, rescue helicopters, pharmacies, or the like.

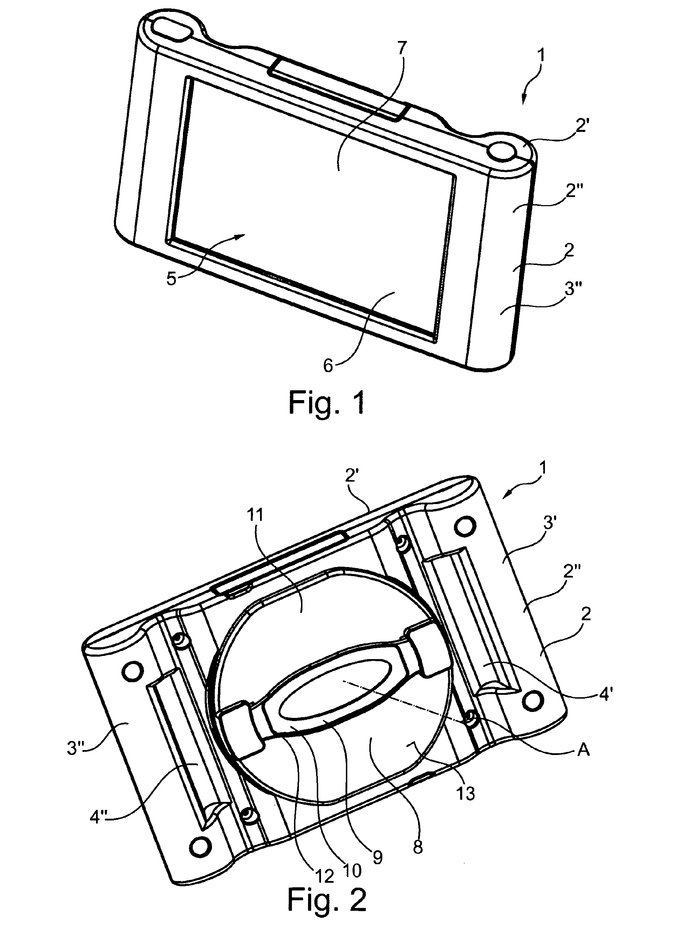

Electrocardiograph 1 comprises a housing 2, preferably shaped to enable an user to comfortably grip the electrocardiograph 1. Specifically, housing 2, made for example in a plastics material, can be substantially shaped as a parallelepiped having rounded ends 3' and 3", preferably along the shortest size of the parallelepiped, in order to make easier to grip it by the user hands. Elongated recesses 4' and 4" can be formed on a side of the shell near the rounded ends 4' and 4", respectively, to enable the insertion of the fingertips and therefore ensure a safe grip by the user (Figure 2). Housing 2 advantageously defines inside a recess 29 adapted to receive the components necessary for the electrocardiograph operation, such as a control processor, memory elements, electrical connections, batteries, etcetera. According to a possible embodiment, housing 2 comprises a first half shell 2' and second half shell 2" connected each other. In this way, for entering recess 29 of housing 2, it is only required to separate one half shell from the other.

Electrocardiograph 1 comprises means for detecting the heart-beat of a patient (not shown in the figures). Particularly, such heart-beat detecting means comprises electrodes to be applied on the patient, connectable to the electrocardiograph 1 in a wired or wireless mode.

In addition to the electrodes, electrocardiograph 1 can comprise further means for detecting further patient parameters, which, in an exemplifying way, can comprise:
- means for detecting the saturation;
- means for detecting the arterial pressure;
- means for detecting the respiratory flow and volume;
- means for measuring the body weight;
- means for measuring the temperature.

In addition, further peripheral devices can be associated to the electrocardiograph 1, such as for example a GPS receiver, a smartcard reader, a fingerprints reader. Such peripheral devices can be connected to the electrocardiograph 1 by a wired or wireless mode (for example Bluetooth or Zigbee).

The data acquired by the above mentioned means can be transmitted to an outside platform, which in turn can transmit pre-existing data to the electrocardiograph 1, by a wired or wireless mode (for example GSM, GPRS, or Wi-Fi).

In order to ensure the operation also without external power sources, the electrocardiograph 1 preferably comprises rechargeable batteries (not shown in the figures).

Electrocardiograph 1 comprises interface-communication means 5 adapted to exchange information and/or commands between the electrocardiograph 1 and an user or the patient himself/herself (Figure 1). According to a possible embodiment, such interface-communication means 5 comprise a touch screen 6 which simultaneously enables to read information and input data or instructions. For example, screen 6 can display a virtual keyboard (not shown in the figures) by which the patient data are input, or virtual keys for providing commands regarding the implementation of an electrocardiogram (start, stop, etcetera). Moreover, the screen 6 can display the results of the performed measurements, for example, the performed electrocardiogram.

Obviously, as an alternative to the touch screen, different kinds or interface-communication means can be provided. For example, it is possible to provide a non touch screen and an independent real keyboard.

Preferably, the interface-communication means 5 are connected to a first face 7 of the electrocardiograph 1 (Figure 1), while the above mentioned elongated fingers receiving recesses 4', 4" are located on a second face 8 opposite to the first face 7 (Figure 2).

Electrocardiograph 1 comprises a gripping-and-supporting member 9, preferably a handle 10, so that the electrocardiograph can be gripped, raised, and transported by an user or also by the patient subjected to the electrocardiogram (Figures 2-4). Such gripping-and-supporting member 9 is rotatably connected to the housing 2. In this way, it is possible to rotate, by modifying the orientation, the electrocardiograph 1 by firmly gripping it by the gripping-and-supporting member 9. Therefore, the use of the electrocardiograph 1 is easy and adaptable to the conditions of use. For example, in case it were necessary to input data, such operation would be simple because one hand would firmly grip the gripping-and-supporting member 9, while the other hand would operate on the touch screen 6, rotating the housing 2 when desired.

Preferably, the gripping-and-supporting member 9 is arranged on the second face 8, opposite to the first face 7, on which the screen 6 is positioned (Figure 2). In this way, the user can support the electrocardiograph 1 from the back (second face 8) and can operate on the screen 6 from the front side (first face 7), rotating the electrocardiograph 1 as desired, by acting for example on the housing 2 keeping meantime firmly grasped the gripping-and-supporting member 9.

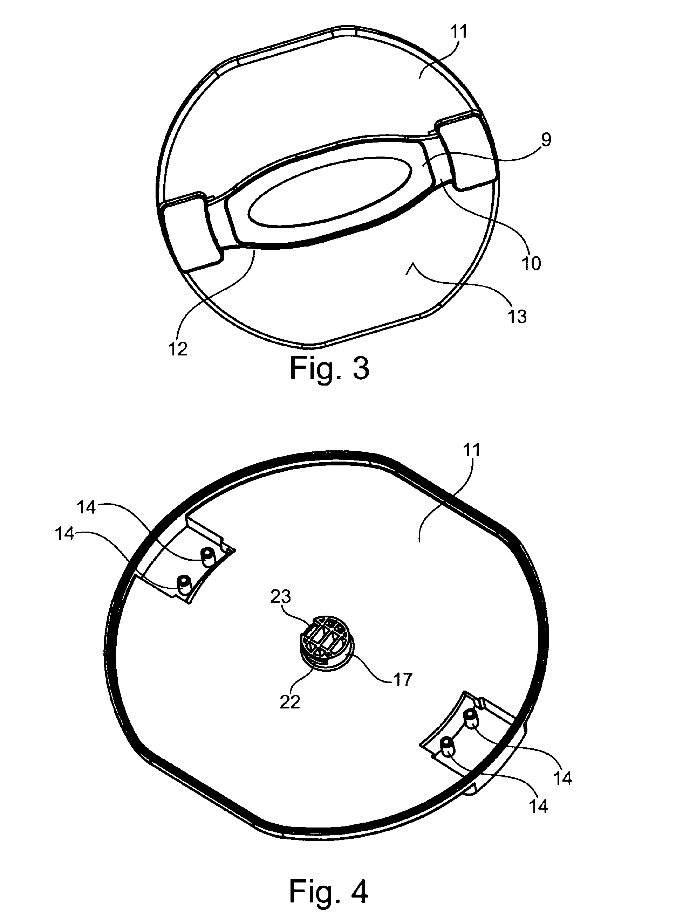

According to a possible embodiment, handle 10 is associated to a table 11, having for example a disc shape, rotatable with respect to the housing 2 (Figures 2 and 3). Specifically, table 11 is rotatably connected to the housing 2 at the second face 8. Such rotatable connection, whose modes of implementation will be described in the following, is preferably embodied so that the table 11 rotates with respect to the housing 2 around a rotation axis A perpendicular to the second face 8.

Advantageously, the handle 10 and table 11 define a space 12 which at least partially receive the hand of an user in a substantially stretched condition. The space has a shape and size such that, when the hand is inserted inside it, the hand palm directly rests on table 11, particularly on its rest surface 13, preferably flat, and the hand back is at least partially wrapped by the handle 10. In this way, the user can support by a hand, inserted in space 12, the whole electrocardiograph 1, and with the other hand can easily rotate the housing with respect to the table 11 and operate on the screen 6.

In order to connect the handle 10 to the table 11, the latter can comprise, on its inner side (in other words on the side facing the opening defined by the housing 2) one or more pins 14 located in diametrally opposed positions, inserted in respective openings 15 positioned at the ends of the handle 10 (Figure 4). The relative clamping between handle 10 and table 11 can be performed for example by washers (not shown in figures) clamped by screws (which also are not shown) threaded in pins 14.

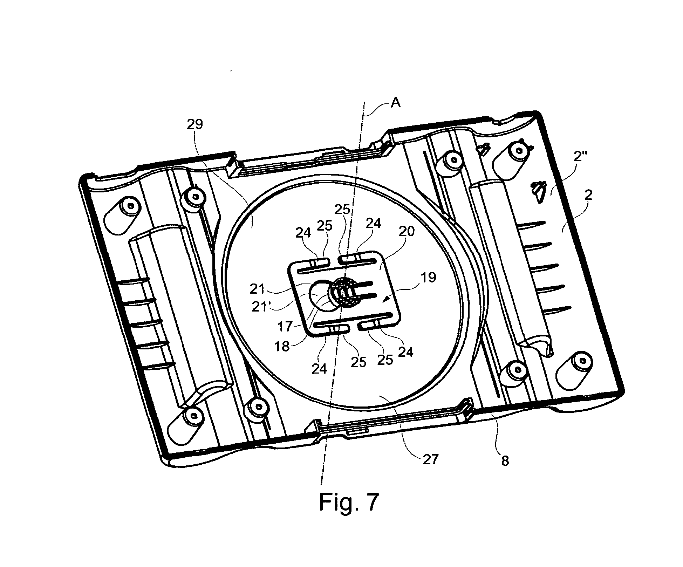

In order to obtain the rotating connection between table 11 and housing 2, particularly the second half shell 2", advantageously, the table 11 comprises a projecting pin 17, and housing 2 comprises a through hole 18 which receives pin 17 (Figure 7). The pin 17 of table 11 is rotatable in hole 18 of housing 2 according to the rotation axis A, coinciding with the axes of pin 17 and hole 18. The cross-section of pin 17 and hole 18 can for this matter have a circular shape.

More advantageously, in order to avoid pin 17 from unthreading from the through hole 18, particularly along the rotation axis A, the electrocardiograph 1 comprises means 19 for axially clamping the pin 17 in hole 18.

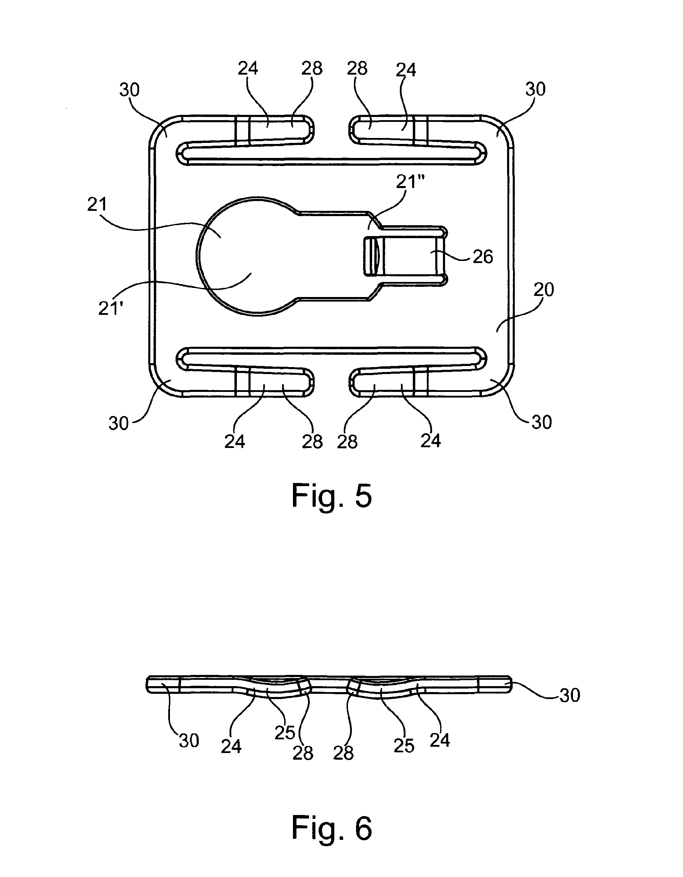

According to an embodiment, the axial clamping means 19 comprises a removable plate 20 integrally translationally connected to the pin 17 and resting on a wall 27 of housing 2 on the inner side of a second face 8 of the latter (Figures 5-7).

Plate 20 comprises a through opening 21 having a first portion 21' having a shape and extension so that pin 17 of table 11 can enter inside it, and a second portion 21" having an extension shorter than the extension of the first portion 21', so that pin 17 cannot be inserted inside it. However, advantageously, pin 17 comprises a notch 22 which can receive the edges of plate 2, which define the second portion 21" of opening 21. When the notch 22 is engaged with the edges of the second portion 21" of the opening 21, the pin 17 of the table 11 is axially bound to housing 2. Indeed, pin 17 cannot unthread from through hole 18 to the outside since plate 10 rests on wall 27 on the inner side of housing 2, and to the inside since table 11 rests on the same wall 27 on the outside side of housing 2.

Advantageously, in order to prevent plate 20 from incidentally disengaging from the side notch 22 of pin 17, plate 11 comprises a resilient latching tag 26, preferably in a central position in plate 20 and partially cantilevered inside the second portion 21" of opening 21. Such latching tag 26 is adapted to snap into a corresponding latching seat 23 formed at an end of pin 17 of table 11. Plate 20, according to this coupling mode, is translationally bound to pin 17 in a transversal direction, particularly perpendicular to the rotation axis A, coinciding with the pin 17 axis. Therefore, the plate is prevented from translating along housing 2 wall 27, so that pin 17 is prevented from entering the first portion 21' of the opening 21 of the plate and therefore the pin 17 is prevented from unthreading from hole 18.

In order to further improve the axial anvil action exerted by plate 20, advantageously the latter comprises one or more further resilient anvil tags 24 resting on housing 2 wall 27, adapted to bias plate 20 away from table 11 and therefore adapted to bring table 11 near the same wall 27, on the outer side. According to the embodiment shown in Figures 5 and 6, plate 20 comprises four of these resilient anvil tags 24, arranged in pair on the two opposite sides of plate 20. According to such embodiment, anvil tags 24 are bound to plate 20 at the respective outer ends 30 of each side, and have free ends 28 facing one the other in each pair of anvil tags 24. Advantageously, the resilient anvil tags 24 comprise curved portions 25, preferably at the free ends 28, convex towards the wall 27 and contacting the same. In this way, in an assembled condition, such curved portions 25 cause the anvil tags 24 to slightly bend, which in turn generates an elastic force pressing the anvil tags against the inner wall 27 of housing 2.

The assembly of table 11 to housing 2 is done in the following way. Table 11 is taken near the second face 8 of housing and pin 17 of table 11 is inserted in housing 2 hole 18 from the outer side. A portion of pin 17 therefore projects on the inner side of the second face 8 of housing 2. Plate 20 is therefore fit on such projecting portion of pin 17, which is inserted in first portion 21' of opening 21 along rotation axis A. Therefore, plate 20 is translated in a direction orthogonal to the rotation axis A, so that pin 21 occupies the second portion 21" of opening 21, whose edges engage the notch 22 of pin 17. Following such translation of plate 20, the resilient clamping tag 26 of plate 20 snaps into the engagement seat 23 of pin. Therefore, table 11 is rotatably coupled along the rotation axis A to housing 12 since it is axially bound along such rotation axis A by plate 20.

Advantageously, the electrocardiograph 1 comprises sensors (not shown in figures) adapted to determine the housing 2 orientation. For example, such sensors can comprise accelerometers or inertial sensors. More advantageously, the screen 6 is configured so that an image or information displayed on it rotates as a function of the housing 2 orientation detected by the orientation sensors. In this way, according to the orientation of housing 2 which can be rotated with respect to table 11 with the described modes, an optimal readout of the information made available on the screen is ensured to the user.

From the above description, a person skilled in the art can appreciate how the electrocardiograph according to the invention, thanks to the rotatable coupling between the housing and the gripping-and-supporting member, is easily and comfortably useable also in arduous positions, both for reading the supplied data and inputting information. Indeed, by one hand it is possible to support the electrocardiograph by the gripping-and-support member, while with the other hand it is possible to operate on the electrocardiograph (for example inserting data by the touch screen) by rotating the housing when desired, to locate it in an optimal position.

A person skilled in the art in order to fulfill specific contingent needs can introduce several variations, additions, or substitutions of elements with other functionally equivalent to the described embodiments, without departing from the scope of the attached claims.

## Claims

1. Portable electrocardiograph (1) comprising:
- a housing (2);
- means for detecting the heart-beat of a patient;
- interface-communication means (5) adapted to exchange information and/or commands between a user or a patient and the electrocardiograph (1);
- a member (9) for gripping and supporting the electrocardiograph (1) by the user or patient,
wherein said gripping and supporting member (9) is rotatably connected to the housing (2),
**characterized in that** said housing (2) comprises a first face (7), in which the interface-communication means (5) are at least partially arranged, and a second face (8) opposite to the first one in which said gripping and supporting member (9) is arranged, wherein said gripping and supporting member (9) is rotatable around a rotation axis (A) perpendicular to the second face (8) of the housing (2).

2. Portable electrocardiograph (1) according to claim 1, wherein said gripping and supporting member (9) is associated to a table (11) rotatably connected to the housing (2).

3. Portable electrocardiograph (1) according to the preceding claim, wherein said gripping and supporting member (9) and said table (11) are configured so to define a space (12) in which a hand of the user or patient can be at least partially substantially stretchedly inserted, with the palm of the hand resting on the table (11) and the back of the hand being wrapped by the gripping and supporting member (9).

4. Portable electrocardiograph (1) according to claim 2 or 3, wherein said table (11) comprises a projecting pin (17) and said housing (2) comprises a through hole (18) receiving at least partially said pin (17), pin (17) of table (11) being rotatable in the housing (2) hole (18).

5. Portable electrocardiograph (1) according to the preceding claim, comprising means (19) for axially blocking the table pin (17) in the housing (2) hole (18).

6. Portable electrocardiograph (1) according to the preceding claim, wherein said axial blocking means (19) comprise a removable plate (20) restingly arranged on an inner wall (27) of the housing (2) and integrally translationally connected to table (11) pin (17), in order to prevent the table (11) pin (17) from outwardly slipping out from the housing (2) hole (18).

7. Portable electrocardiograph (1) according to the preceding claim, wherein said plate (20) comprises a through opening (21) in which the table (11) pin (17) is partially inserted, said through opening (21) comprising a first portion (21') having an extent greater than or equal to the extent of the cross-section of the table (11) pin (17), and a second portion (21") having an extent less than the extent of the first portion (21'), said pin (17) having further a side notch (22) such to define an axial portion of the pin, having a reduced cross-section in which the plate (20) is inserted at edges defining said second portion (21') of the through opening (21).

8. Portable electrocardiograph (1) according to the preceding claim, wherein said plate (20) comprises a resilient latching tag (26) adapted to snap into a corresponding latching seat (23) of the table (11) pin (17) in order to prevent the table (11) pin (17) and the plate (20) from translating one from the other, which would take the pin (17) to locate itself at the first portion (21') of the plate (20) through opening (21).

9. Portable electrocardiograph (1) according to anyone of claims 6-8, wherein said plate (20) comprises one or more resilient anvil tags (24) abutting said inner wall (27) of housing (2), adapted to bias the plate (20) away from the table (11).

10. Portable electrocardiograph (1) according to the preceding claim, wherein said resilient anvil tags (24) comprise curved portions (25) convex towards said inner wall (27) of housing (2) and in contact with it.

11. Portable electrocardiograph (1) according to anyone of the preceding claims, comprising orientation sensors adapted to detect the orientation of the housing (2), wherein said interface-communication means (5) comprise a screen (6) configured so that information and/or images provided therefrom modify their orientation as a function of the housing (2) orientation detected by said orientation sensors.

## Patentansprüche

1. Tragbarer Elektrokardiograph (1), umfassend:
- ein Gehäuse (2);
- eine Einrichtung zur Erfassung des Herzschlags eines Patienten;
- eine Schnittstellen-Kommunikationseinrichtung (5), die dafür ausgelegt ist, Informationen und/oder Befehle zwischen einem Anwender oder einem Patienten und dem Elektrokardiographen (1) auszutauschen;
- ein Element (9) zum Greifen und Tragen des Elektrokardiographen (1) durch den Anwender oder Patienten,
wobei das Greif- und Tragelement (9) drehbar mit dem Gehäuse (2) verbunden ist,
**dadurch gekennzeichnet, dass** das Gehäuse (2) eine erste Fläche (7), auf der zumindest zum Teil die Schnittstellen-Kommunikationseinrichtung (5) angeordnet ist, und eine zweite Fläche (8) entgegengesetzt zur ersten aufweist, auf der das Greif- und Tragelement (9) angeordnet ist, wobei das Greif- und Tragelement (9) um eine Drehachse (A) drehbar ist, die senkrecht ist zur zweiten Fläche (8) des Gehäuses (2).

2. Tragbarer Elektrokardiograph (1) nach Anspruch 1, wobei das Greif- und Tragelement (9) einer Scheibe (11) zugeordnet ist, die drehbar mit dem Gehäuse (2) verbunden ist.

3. Tragbarer Elektrokardiograph (1) nach dem vorangehenden Anspruch, wobei das Greif- und Tragelement (9) und die Scheibe (11) so gestaltet sind, dass sie einen Zwischenraum (12) definieren, in den eine Hand des Anwenders oder Patienten zumindest zum Teil im Wesentlichen ausgestreckt eingeführt werden kann, wobei die Handfläche auf der Scheibe (11) ruht und sich das Greif- und Tragelement (9) um den Handrücken legt.

4. Tragbarer Elektrokardiograph (1) nach Anspruch 2 oder 3, wobei die Scheibe (11) einen vorstehenden Zapfen (17) umfasst und das Gehäuse (2) eine durchgehende Bohrung (18) umfasst, die den Zapfen (17) zumindest zum Teil aufnimmt, wobei der Zapfen (17) der Scheibe (11) in dem Loch (18) des Gehäuses (2) drehbar ist.

5. Tragbarer Elektrokardiograph (1) nach dem vorangehenden Anspruch, eine Einrichtung (19) zum axialen Blockieren des Zapfens (17) der Scheibe in dem Loch (18) des Gehäuses (2) umfassend.

6. Tragbarer Elektrokardiograph (1) nach dem vorangehenden Anspruch, wobei die Einrichtung (19) zum axialen Blockieren eine abnehmbare Platte (20) umfasst, die an einer Innenwand (27) des Gehäuses (2) aufliegend angeordnet ist und integral translational mit dem Zapfen (17) der Scheibe (11) verbunden ist, um zu verhindern, dass der Zapfen (17) der Scheibe (11) auswärts aus dem Loch (18) des Gehäuses (2) rutscht.

7. Tragbarer Elektrokardiograph (1) nach dem vorangehenden Anspruch, wobei die Platte (20) eine durchgehende Öffnung (21) umfasst, in welche der Zapfen (17) der Scheibe (11) zum Teil eingeführt wird, wobei die durchgehende Öffnung (21) einen ersten Abschnitt (21') mit einer Abmessung, die größer oder gleich der Abmessung des Querschnitts des Zapfens (17) der Scheibe (11) ist, und einen zweiten Abschnitt (21 ") aufweist, der eine Abmessung aufweist, die kleiner ist als die Abmessung des ersten Abschnitts (21'), wobei der Zapfen (17) ferner eine seitliche Kerbe (22) aufweist, um einen axialen Abschnitt des Zapfens zu definieren, der einen verkleinerten Querschnitt aufweist, in den die Platte (20) an Rändern, die den zweiten Abschnitt (21') der durchgehenden Öffnung (21) definieren, eingeführt wird.

8. Tragbarer Elektrokardiograph (1) nach dem vorangehenden Anspruch, wobei die Platte (20) eine elastische Verriegelungslasche (26) umfasst, die dafür ausgelegt ist, in einen entsprechenden Verriegelungssitz (23) des Zapfens (17) der Scheibe (11) einzurasten, um zu verhindern, dass der Zapfen (17) der Scheibe (11) und die Scheibe (20) sich auseinander verschieben, wodurch der Zapfen (17) sich selbst am ersten Abschnitt (21') der durchgehenden Bohrung (21) der Platte (20) anordnen würde.

9. Tragbarer Elektrokardiograph (1) einem der Ansprüche 6-8, wobei die Platte (20) eine oder mehrere elastische Anschlagslaschen (24) aufweist, die an der Innenwand (27) des Gehäuses (2) anliegen und dafür ausgelegt sind, die Platte (20) von dem Scheibe (11) weg zu drängen.

10. Tragbarer Elektrokardiograph (1) nach dem vorangehenden Anspruch, wobei die elastischen Anschlagslaschen (24) gekrümmte Abschnitte (25) aufweisen, die sich gegen die Innenwand (27) des Gehäuses (2) vorwölben und damit in Kontakt stehen.

11. Tragbarer Elektrokardiograph (1) nach einem der vorangehenden Ansprüche, Ausrichtungssensoren umfassend, die dafür ausgelegt sind, die Ausrichtung des Gehäuses (2) zu fühlen, wobei die Schnittstellen-Kommunikationseinrichtung (5) einen Bildschirm (6) umfasst, der so gestaltet ist, dass Informationen und/oder Bilder, die davon wiedergegeben werden, ihre Ausrichtung als Funktion der von den Ausrichtungssensoren erfassten Ausrichtung des Gehäuses (2) modifizieren.

## Revendications

1. Électrocardiographe portable (1) comprenant :
- un boîtier (2) ;
- des moyens pour détecter le battement de coeur d'un patient ;
- des moyens d'interface-communication (5) aptes à échanger des informations et/ou des instructions entre un utilisateur ou un patient et l'électrocardiographe (1) ;
- un élément (9) pour une saisie et un support de l'électrocardiographe (1) par l'utilisateur ou le patient,
ledit élément de prise et de support (9) étant relié de façon rotative au boîtier (2),
**caractérisé par le fait que** ledit boîtier (2) comprend une première face (7), dans laquelle les moyens d'interface-communication (5) sont au moins partiellement agencés, et une seconde face (8) opposée à la première face, dans laquelle ledit élément de prise et de support (9) est agencé, ledit élément de prise et de support (9) étant apte à tourner autour d'un axe de rotation (A) perpendiculaire à la seconde face (8) du boîtier (2).

2. Électrocardiographe portable (1) selon la revendication 1, dans lequel ledit élément de prise et de support (9) est associé à une table (11) reliée de façon rotative au boîtier (2).

3. Électrocardiographe portable (1) selon la revendication précédente, dans lequel ledit élément de prise et de support (9) et ladite table (11) sont configurés de façon à définir un espace (12) dans lequel une main de l'utilisateur ou du patient peut être au moins partiellement introduite de manière sensiblement étendue, avec la paume de la main reposant sur la table (11) et le dos de la main enveloppé par l'élément de prise et de support (9).

4. Électrocardiographe portable (1) selon la revendication 2 ou 3, dans lequel ladite table (11) comprend un pivot en saillie (17), et ledit boîtier (2) comprend un trou traversant (18) recevant au moins partiellement ledit pivot (17), le pivot (17) de la table (11) étant apte à tourner dans le trou (18) du boîtier (2).

5. Électrocardiographe portable (1) selon la revendication précédente, comprenant des moyens (19) pour bloquer axialement le pivot de table (17) dans le trou (18) du boîtier (2).

6. Électrocardiographe portable (1) selon la revendication précédente, dans lequel lesdits moyens de blocage axial (19) comprennent une plaque amovible (20) agencée à appui sur une paroi intérieure (27) du boîtier (2) et reliée à translation, d'un seul tenant, au pivot (17) de la table (11), afin d'empêcher le pivot (17) de la table (11) de glisser vers l'extérieur hors du trou (18) du boîtier (2).

7. Électrocardiographe portable (1) selon la revendication précédente, dans lequel ladite plaque (20) comprend une ouverture traversante (21) dans laquelle le pivot (17) de la table (11) est partiellement introduit, ladite ouverture traversante (21) comprenant une première partie (21') ayant une étendue supérieure ou égale à l'étendue de la section transversale du pivot (17) de la table (11), et une seconde partie (21") ayant une étendue inférieure à l'étendue de la première partie (21'), ledit pivot (17) ayant en outre une encoche latérale (22) de façon à définir une partie axiale du pivot, ayant une section transversale réduite dans laquelle la plaque (20) est introduite au niveau de bords définissant ladite seconde partie (21') de l'ouverture traversante (21).

8. Électrocardiographe portable (1) selon la revendication précédente, dans lequel ladite plaque (20) comprend une languette de verrouillage élastique (26) apte à s'encliqueter dans un siège de verrouillage (23) correspondant du pivot (17) de la table (11) afin d'empêcher le pivot (17) de la table (11) et la plaque (20) de se déplacer en translation l'un à l'opposé de l'autre, ce qui amènerait le pivot (17) à se situer lui-même à la première partie (21') de l'ouverture traversante (21) de la plaque (20).

9. Électrocardiographe portable (1) selon l'une quelconque des revendications 6 à 8, dans lequel ladite plaque (20) comprend une ou plusieurs languettes de choc élastiques (24) contiguës à ladite paroi intérieure (27) du boîtier (2), aptes à solliciter la plaque (20) à l'opposé de la table (11).

10. Électrocardiographe portable (1) selon la revendication précédente, dans lequel lesdites languettes de choc élastiques (24) comprennent des parties incurvées (25) convexes vers ladite paroi intérieure (27) du boîtier (2) et en contact avec celle-ci.

11. Électrocardiographe portable (1) selon l'une quelconque des revendications précédentes, comprenant des capteurs d'orientation aptes à détecter l'orientation du boîtier (2), lesdits moyens d'interface-communication (5) comprenant un écran (6) configuré de telle sorte que des informations et/ou des images fournies à partir de celui-ci modifient leur orientation en fonction de l'orientation du boîtier (2) détectée par lesdits capteurs d'orientation.
